# EUROPEAN PATENT SPECIFICATION

(11) **EP 1 126 891 B1**
(45) Date of publication and mention of the grant of the patent: **17.12.2003**
(21) Application number: 99956676.3
(22) Date of filing: 25.10.1999
(51) Int. Cl.: A61M 15/00, A61K 9/00, B05D 1/04

(54) **METHOD OF DEPOSITING A DRY POWDER AND DISPENSING DEVICE**
VERFAHREN ZUM AUFTRAGEN VON TROCKENPULVER SOWIE VERABREICHUNGSVORRICHTUNG
METHODE DE DEPOT D'UNE POUDRE SECHE ET DISTRIBUTEUR

(30) Priority: 05.11.1998 US 187092
(43) Date of publication of application: 29.08.2001
(73) Proprietor: Delsys Pharmaceutical Corporation, Monmouth Junction, NJ 08852 (US)
(72) Inventor: CHRAI, Suggy, S., Cranbury, NJ 08512 (US); MCGINN, Joseph, Thomas, Flemington, NJ 08822 (US); SINGH, Bawa, Voorhees, NJ 08043 (US)
(74) Representative: Pratt, Richard Wilson
(86) International application number: PCT/US99/25051
(87) International publication number: WO 00/027456

(56) References cited:
- WO-A-93/09832
- US-A- 5 714 007

## Description

This invention relates to a method of depositing dry powders on a substrate, and in particular, medicaments for use with inhalers, for example, and inhaler devices for use with such substrates.

Cross Reference to Related Applications and Patents Of interest are co-pending applications Serial No. 08/661,213 entitled Inhaler Apparatus with Modified Surfaces for Enhanced Release of Dry Powders filed June 10, 1996 in the name of Datta et al. now US Pat. No. 5,871,010, Inhaler Apparatus with an Electronic Means for Enhanced Release of Dry Powders Serial No. 08/661,212 filed June 10, 1996 in the name of Sun et al. now US Pat. No. 5,857,456, Serial No. 08/932,489 entitled Dry Powder Delivery System filed

September 18, 1997 in the name of Leedom et al, now US Patent No. 6,237,590, Serial No. 08/659,501 entitled Methods and Apparatus for Electrostatically Depositing a Medicament Powder Upon Predefined Regions of a Substrate filed June 6, 1996 in the name of Pletcher et al, now US Patent No. 6,007,630; Serial No. 09/095,246 entitled Dry Power Deposition Process filed June 10, 1998 in the name of Poliniak et al. now US Patent No. 6,063,194; Serial No. 09/095,616 entitled Pharmaceutical Product and Method of Making filed June 10, 1998 in the name of Chrai et al. now US Patent No. 6,303, 143; and US Patents Nos. 5,714,007, 5,642,727, and 5,669,973 commonly owned with the aforementioned applications.

Dry powder inhalers are used as drug delivery devices for pharmaceutical compounds to individuals. In these devices, a pharmaceutical powder is deposited on a surface of a substrate. The substrate may then be supplied in the inhaler as a cassette, a cartridge and so on. When the patient requires medication, the ideal dry powder inhaler forms a fine particle cloud that is to be inhaled and thereby delivers a high respirable fraction of the stored dose deeply into the patients lungs. In most cases, the deep recesses of the lung is the desired site for the drugs in the inhaled powder cloud.

This can be most efficiently achieved by:
1. Releasing a high fraction of the deposited drug and
2. Insuring that the powder cloud consists of individual particles or particle aggregates between 1µm and 5µm.

As individual particles are reduced below 10µm, both release and particle aggregation become serious hindrance to delivering a high respirable fraction deeply into the patient's lungs.

A common problem dealt with by various prior art inhaler apparatuses for dispensing dry powder medicaments is providing for a controlled reliable release of the medicament. The dry powder inhalers may be loaded with medicaments by filling techniques not involving electrostatics. In certain implementations, the deposited powder tends to form agglomerated particles resulting in uncontrolled variation in the amount of medicament released. Several of the aforementioned applications provide various solutions to this problem.

Numerous approaches have been taken in the design of dry powder inhalers. In some cases, the powder is released by impact of a substrate powder carrier, as disclosed in WO 93/09832. Of interest is an inhaler as disclosed in WO 90/13328.

In the patent specifications US-A-5 871 010 aud US-A-5 857 456, indentations or raised surfaces are disclosed in the inhaler interior surfaces having contact with the medicament for inhalation, the surfaces minimizing the area of contact between the medicament and the surfaces of the inhaler apparatus, promoting the release of the medicament from the inhaler.

Where particles of medicament agglomerate, they impact the mouth and throat rather than remain in the air flow for deposition in the lungs. One remedy is to provide tortuous channels in the inhalers to promote deagglomeration. However, the medicament may be deposited along the channels leading to inaccurate dosage dispensing. Agglomeration also results in the inhaler tending to dispense the medicament inaccurately so that greater or lesser amounts are dispensed. The aforementioned solutions to the problem of agglomeration tend to rely on mechanical devices for minimising agglomeration effects.

The small particle size required for transport to the lung presents a number of problems for release by the inhaler and delivery to deep lung regions. As the particle size decreases, the relative bonding force between the particle and other objects increases. This applies to both particle-to-substrate bonding and particle-to-particle bonding. As a result, particle aggregates become more tightly bound and individual particles are more difficult to remove from the substrate. Aggregation increases the effective size of the drug released and diminishes the respirable fraction. The increase in relative particle-to-substrate bonding makes drug release more difficult and also decreases the respirable fraction.

The present inventors recognise a need for a different approach wherein the agglomeration itself is minimised so as to be negligible. This reduces the need for mechanical solutions to deal with the agglomeration effects.

Respective aspects and features of the present invention are set out in the claims.

A method of depositing dielectric elongated pharmaceutical active medicament dry powder particles on a substrate according to an embodiment of the present invention comprises providing a plurality of elongated dry particles each having a longitudinal axis and depositing the particles on a surface of a substrate with the longitudinal axes aligned substantially normal to the substrate surface.

Preferably the method includes in one embodiment charging the particles with a given polarity and electrostatically depositing the particles on the substrate.

In a further embodiment, the particles are provided with an aspect ratio such that an electrical dipole is created in the particles along the axis. An electrical field attracts the particles to the substrate.

In a still further preferred embodiment, the particles have an aspect ratio of about 2:1.

The substrate may be metallic, or non-metallic including mesh and non-mesh materials.

In a further embodiment, a controlled amount of the medicament is deposited at each of a plurality of predetermined regions on the surface of the substrate.

The particles in a further preferred embodiment have a diametrical dimension in the range of about 0.5 µm to 3 µm and a length in the range of about 1 µm to 10 µm.

The particles are a pharmaceutically active ingredient medicament.

A device for dispensing a pharmaceutical drug according to a further embodiment of the present invention comprises an inhaler having a mouthpiece and a medicament cavity in communication with the mouthpiece. A dry powder medicament deposited in discrete spaced locations on a substrate is introduced into the cavity for selective dispensing by the inhaler, the medicament comprising a plurality of elongated particles, the particles having an aspect ratio such as to create an electrical dipole in the particles when charged or induced by the depositing field.

A device according to a still further embodiment of the present invention for depositing a dry powder on a substrate comprises a plurality of elongated dry particles each having a longitudinal axis and means for depositing the particles on a surface of a substrate with the longitudinal axes aligned substantially normal to the substrate surface.

### IN THE DRAWING:

FIGURE 1 is a schematic diagram illustrating in general form a deposition process for depositing particles according to an embodiment of the present invention;
FIGURE 2 is a more detailed view of the diagram of Fig. 1 showing field generation on the substrate.
FIGURES 3 and 4 are electronmicrographs illustrating particles deposited on a substrate according to an embodiment of the present invention;
FIGURE 5 is a side sectional elevation view of an exemplary inhaler incorporating a medicament deposited according to the present invention; and
FIGURES 6 and 6a are side elevation fragmented sectional views of a representative substrate employed in an inhaler apparatus of an embodiment of the present invention.

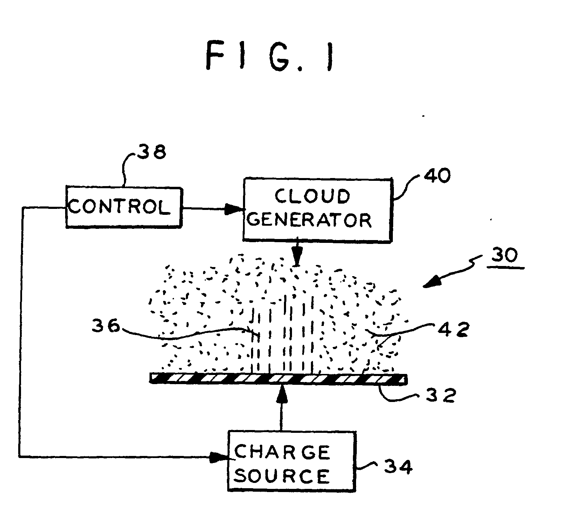

In Fig. 1, deposition system 30 is diagrammatically shown and comprises a substrate 32, a charge source 34 for generating a field represented by dashed lines 36, a control 38 and a medicament cloud generator 40 for generating a cloud of elongated medicament particles 42. Generally electrostatic deposition systems are known as described in the aforementioned copending applications and patents. However the disclosed systems may be modified as described herein according to the present invention.

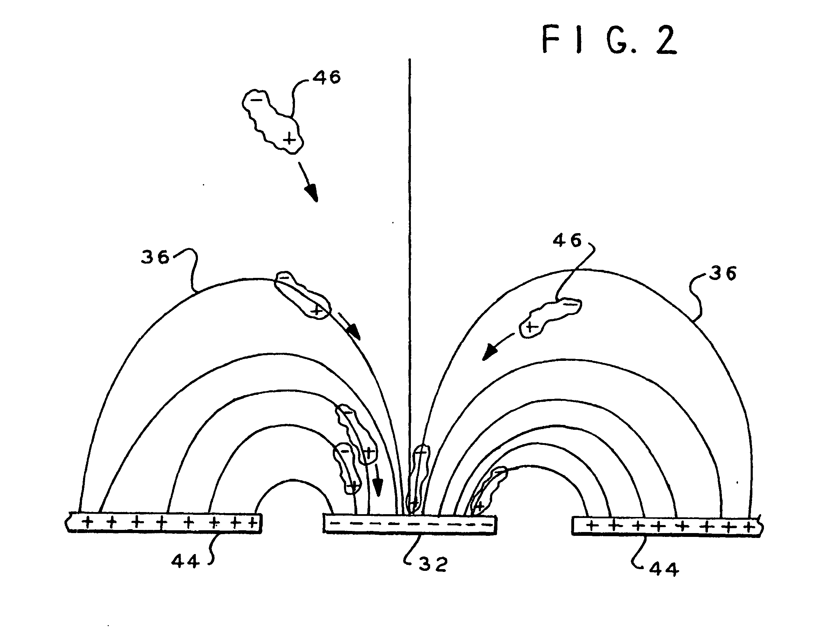

In diagrammatic Fig.2, in one embodiment, the substrate 32 is at one polarity, e.g., negative charged, and a field electrode 44 is at the opposite polarity, e.g., positive charged. Electrode 44 may surround the substrate 32. Substrate 32 is charged at a local region for receiving the medicament in controlled amounts as described for example in the aforementioned patents and copending applications mentioned in the introductory portion. Field lines 36 are created between the substrate 32 and the field electrode 44 as shown. Elongated particles 46 in the field exhibiting dipoles align with the field lines 36 and wherein the charges on the substrate 32 attract the particles 46 to the substrate 32.

The medicament powder is in the form of elongated particles having an approximate diameter preferably in the range of about 0.5 to 3 µm and an axial extent of preferably in the range of about 1 to 10 µm. The particles are charged with a given polarity in a conventional charging mechanism such as triboelectric chargers, induction charging and so on. The particles are deposited in controlled amounts wherein the amount of active pharmaceutical ingredients deposited at each of a plurality of locations on the substrate does not vary from a predetermined amount, for example, by more than 15%.

Reference is made to the copending applications Serial No. 09/095,246 entitled Dry Powder Deposition Process filed June 10, 1998 in the name of Poliniak et al. and Serial No. 09/095,616 entitled Pharmaceutical Product and Method of Making filed June 10, 1998 in the name of Chrai et al. noted in the introductory portion. These applications disclose apparatus and processes for electrostatically depositing pharmaceutically active ingredient medicaments on a substrate including charging a dry powder medicament and electrostatically attracting the charged powder particles to a substrate. In particular, the medicament is deposited in controlled amounts at discrete locations on the substrate wherein the amounts deposited do not vary from a predetermined amount by more than 15%, for example. This process is preferred.

However, other processes for electrostatically depositing dry powder medicaments on a substrate are also disclosed in the aforementioned copending applications and patents noted in the introductory portion. Those processes disclose electrostatically depositing controlled amounts of dry powder medicaments on a substrate at discrete locations on the substrate. Variations of the disclosed process herein may be employed to adapt those processes to a metal substrate, whether a tape, mesh or disk with radially extending fingers on which medicaments are deposited as will be described below as employed in the present embodiments. Such variations are within the skill of those of ordinary skill in this art.

In the present invention, elongated dry medicament particles are charged with one polarity, e.g., positive charges and electrostatically deposited on a substrate exposed to an electric field, with a spatial variation arranged to attract the charged particles to discrete predetermined locations on a substrate. This is to be differentiated from prior art processes wherein the particles tend to be generally spherical, amorphous or symmetrical.

Particle removal from surfaces tends to be more difficult as particle size decreases. This is roughly a consequence of the adhesion force decreasing more slowly than the volume and surface area as a particle's size decreases. Since the volume and surface are generally related to removing forces and deaggregation, these forces become increasingly difficult to overcome as the particle size decreases.

Forces of adhesion and agglomeration caused by van der Waal's force increase as the area of contact between a particle and substrate or between two particles increase. If highly elongated particles can be made to deposit on substrates in such a manner that only their tips contact a substrate, adhesion will be lower than if similar particles deposit with their long axes in contact with the substrate. Similarly, agglomeration forces between highly elongated particles will be minimized if they contact tip-to-tip. Agglomeration can also be decreased if the particle density is sufficiently low as to inhibit particle-particle contact along their major axes.

To obtain high respirable fractions, electrostatic deposition is preferred to minimize particle-substrate and particle-particle contact which minimizes adhesive and agglomeration forces respectively. Most if not all pharmaceutical powders are dielectric and can support the separation of electrical charges. In the presence of an electric field, such as the field manifested by lines 36, Fig. 2, particles of such powder tend to become electrically polarized forming dipoles. The particles do not need to be separately charged independently of the field to form such dipoles.

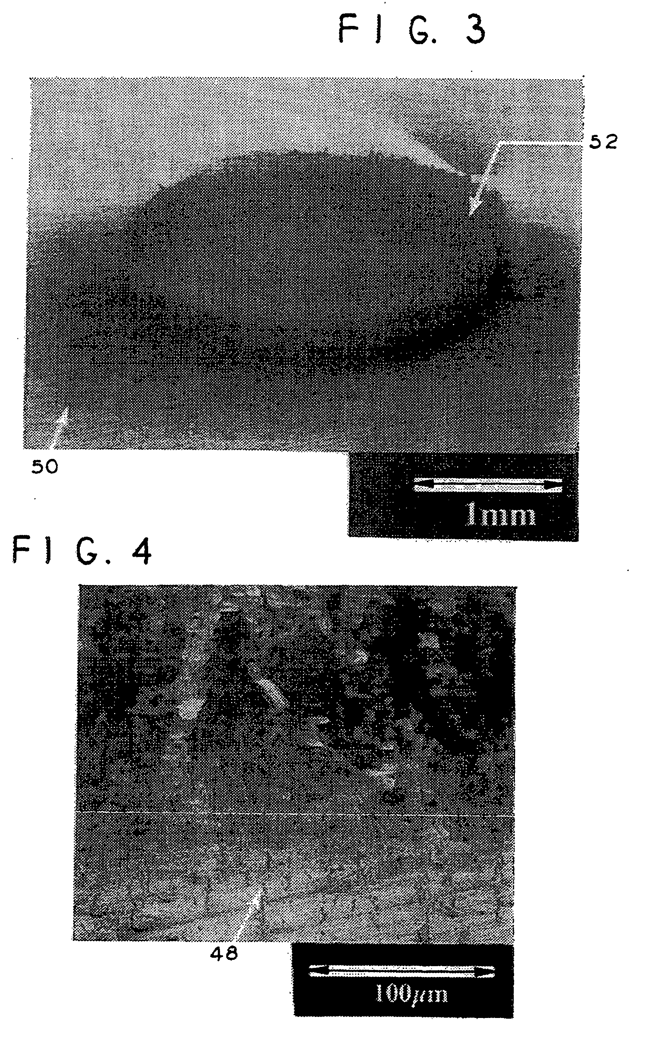

That is, the elongated particles tend to form dipoles with a positive charge at one tip and a negative charge at the opposite tip. A field is applied to the substrate to attract particles charged with a positive polarity, Fig. 2. When the particles are attracted to the substrate by the electric field, the dipole charge in the particles aligns the particles so that their major axis is generally normal to the substrate surface. This attracts the particle tips to the substrate via opposite polarity charges in the substrate field and particle tip. The particles thus stand upright end up on the substrate..Figs. 3 and 4 are electronmicrographs showing this end-to-end configuration. Fig. 3 shows the grains of a deposited pharmaceutical product at a discrete location on an aluminum substrate. The field aperture 50 outline is shown by scattered particles. The field-defined dose 52 has an open structure as shown in Fig. 3. A comparison of the dose size to grain size is shown by the scale in the figure. Fig. 4 shows the columnar structure of the deposited powder. The scale shows this is an enlargement relative to the scale of Fig. 3.

The higher the aspect ratio of the particles, the greater the polarization. In turn, the polarization of highly acicular particles causes an alignment of the particle's major axis with the electric field line. Introduced charges on insulated dielectric particles will dominate the alignment of the particles.

By controlling the field's geometry, it is possible to align the pharmaceutical particles and direct their deposition to particular locations. For a pre-charged particle, a uniform field will align the particle depending upon its charge distribution. For particles in which polarization is induced by an electrostatic field, alignment will be controlled by the particle geometry, i.e., the long axis is always parallel to the field gradient, Fig. 2. Charged particles will tend to follow field lines. Thus it is advantageous to have a high density of field lines terminating in the preferred deposition site.

Figures 3 and 4 show an example of polyethylene glycol (PEG) electrostatically deposited on an aluminum film substrate. The individual particles have an aspect ratio of at least about 2:1 or greater, e.g., 10:1. Once a particle has been deposited, the electrostatic field acts to direct subsequent particles to form elongated chains of tip-to-tip particles. A relative high density of particles on the substrate that may lead to agglomeration of particles along their major axes and may occur under relatively high deposition conditions.

The low-density deposition in which the particle chains remain individually isolated or in single particle thick chains is an ideal structure for release and deaggregation. Individual particles are either bound to the substrate at their tip or to other particles tip-to-tip. In either case, the resultant geometry offers minimum area for van der Waal interaction and subsequently a minimum in adhesive and agglomeration forces.

The density of electrostatic deposited particles is low (i.e., 0.1-0.4 of bulk density) due to the repulsive forces between the particles with the same charge polarity.

For applications requiring doses below approximately 100 µg, depositions may be made on substrate areas of approximately 50 mm² without substantial aggregate formation. Consequently, they are isolated or form single-particle-thick chains with low interparticle van der Waal forces and release from the substrate at much lower forces than otherwise would occur.

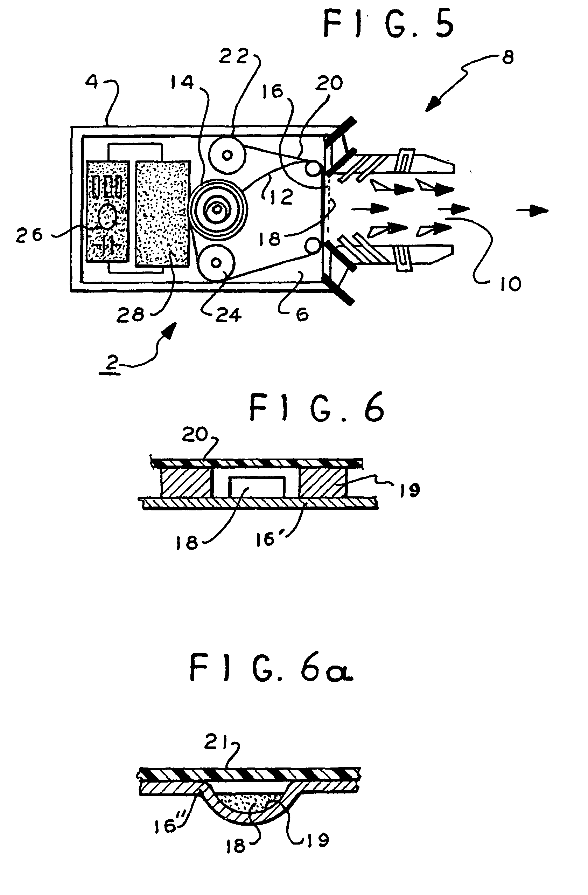

In Figure 5, an inhaler apparatus 2, which is given by way of example in one embodiment, comprises a one-piece molded thermoplastic housing 4 having a chamber 6. Other dispensing devices and apparatuses as known in this art may also be employed for dispensing a dry powder active pharmaceutical ingredient medicament. Such other devices may include cassette or cartridge type dispensers.

A mouthpiece 8 may be molded one-piece with and extends from the housing 4 and has a throat 10 in fluid communication with the chamber 6. A medicament dispensing tape 12 is supplied by reel 14. The tape 12 comprises a metal tape substrate 16, preferably stainless steel. Medicament 18 is deposited on the substrate 16 in controlled amounts at each of a plurality of discrete spaced locations. A tape seal 20 covers and protects the medicament 18 on the substrate 16.

The seal 20 is removed by take up reel 22 and the emptied substrate is taken up by take up reel 24. A battery 26 operates an electronic medicament release mechanism 28 (not shown in detail) but described in copending application Serial No. 661,212 mentioned in the introductory portion and incorporated by reference herein. The inhaler apparatus housing 4, mouthpiece 8, substrate and related mechanisms are described more fully in copending application Serial No. 661, 213 mentioned in the introductory portion and incorporated by reference herein.

The substrates may be employed in cassettes or cartridges and may be either metal, e.g., stainless steel, or non-metallic as known in this art, may be mesh or solid, and may be of any material suitable as a medicament substrate. The selection of a substrate material depends upon a given implementation. In Fig. 6, substrate 16' may comprise a steel tape having a dosage 18 in discrete locations, a spacer 19 and a sealing tape 20. In the alternative, in Fig. 6a, pockets 19, or dimples (not shown) may be formed in the substrate 16". The pockets recesses the dosage 18 and avoids the need for a spacer. This recessing spaces the dosage 18 from the sealing tape 21. When the tape is removed, the dosage remains in place and is not removed by the tape.

It will occur to one of ordinary skill that modifications may be made to the disclosed embodiments without departing from the scope of the invention as defined in the appended claims. For example, the deposited powder may not be a medicament, but may be powders for other applications in accordance with a given implementation. The various substrates are also given by way of illustration. The dispensing device is also by way of example, as other dispensing arrangements may be employed as desired. The description given herein is by way of illustration and not limitation.

## Claims

1. A method of depositing dielectric elongated pharmaceutical active medicament dry powder particles (42) on a substrate (32) comprising:
providing a plurality of dielectric elongated pharmaceutical active medicament dry particles (42) each having a longitudinal axis; and
depositing the particles (42) on a surface of the substrate (32) by providing an electric field (36) which aligns the longitudinal axis of the particles (42) substantially normal to the substrate (32) surface, thereby minimising agglomeration of the particles (42) and minimising adherence of the particles (42) to the substrate (32).

2. The method of claim 1, including charging the particles (42) with a given polarity and electrostatically depositing the particles (42) on the substrate (32).

3. The method of claim 2 including providing the particles (42) with an aspect ratio such that an electrical dipole is created in each particle (42) along its longitudinal axis such that an electrical field (36) electrostatically attracts and aligns the particles (42) to the substrate (32).

4. The method of claim 1, wherein the particles (42) have an aspect ratio of about 2:1.

5. The method of claim 1, including providing a metal substrate (32).

6. The method of claim 5, including providing a stainless steel substrate (32).

7. The method of claim 1, including depositing a controlled amount of the particles (42) at each of a plurality of predetermined regions on the surface of the substrate (32) to form a unit dosage at each said predetermined region.

8. The method of claim 1, including the step of forming particle chains substantially normal to the substrate (32).

9. The method of claim 1, including providing particles (42) with a diametrical dimension in the range of about 0.5 to 3 µm and a length in the range of about 1 to 10 µm.

10. The method of claim 1, including the step of providing a plurality of the particles (42) in a plurality of separate discrete locations on the substrate (32), each location forming a dosage.

11. A device for dispensing a pharmaceutical active medicament (18) comprising:
an inhaler (2) having a mouthpiece (8) and a medicament cavity (6) in communication with the mouthpiece (8); and
a dry powder pharmaceutical active medicament (18) deposited on a substrate (16) in the cavity (6) for selective dispensing by the inhaler (2), **characterized in that**, the medicament (18) comprises a plurality of dielectric dry elongated particles (42), the particles (42) having an aspect ratio such as to create an electrical dipole in the particles (42) when exposed to an applied electrical field (36) the particles (42) being deposited on the substrate (16) with their longitudinal axes substantially normal to the substrate (16) the particles (42) being attracted to the substrate (16) by an electric field (36) to minimise agglomeration and adherence of the particles (42) to the substrate (16).

12. The device of claim 11, wherein the aspect ratio is about 2:1.

13. The device of claim 11, wherein the elongated particles (42) each have opposite end tips, a plurality of the particles (42) contacting the substrate (16) at one of the particle end tips.

14. The device of claim 11, including dispensing means (12, 14) for selective dispensing the medicament.

15. The device of claim 11, wherein a first plurality of the particles (42) engage a second plurality of the particles (42) in tip-to-tip relation.

16. A device (30) for depositing pharmaceutical active medicament dry powder dielectric elongated particles (42) on a substrate (32) comprising:
a plurality of elongated pharmaceutical active medicament dielectric dry particles (42) each having a longitudinal axis; and
means for depositing the particles on a surface of the substrate (32) by providing an electric field (36) which aligns the longitudinal axis of the particles (42) substantially normal to the substrate surface, thereby minimising agglomeration of the particles (42) and minimising adherence of the particles (42) to the substrate (32).

17. The device of claim 16, including means (34) for charging the particles (42) with a given polarity and means (36) for electrostatically depositing the particles on the substrate.

18. The device of claim 16, wherein the particles (42) have an aspect ratio such that an electrical dipole is created in each particle (42) along its longitudinal axis and such that an electrical field (36) electrostatically attracts the particles (42) to the substrate (32).

19. The device of claim 16, wherein the particles (42) have an aspect ratio of about 2:1.

20. The device of claim 16, wherein the substrate (32) is metal.

21. The device of claim 16, including particle chains substantially normal to the substrate (32).

22. The device of claim 16, wherein a plurality of the particles (42) are deposited at each of a plurality of separate discrete locations on the substrate (32) forming a unit dosage at each location.

## Patentansprüche

1. Verfahren zum Abscheiden von dielektrischen, länglichen, pharmazeutisch wirksamen Arzneimitteltrockenpulverpartikeln (42) auf einem Substrat (32), bei dem man
eine Vielzahl dielektrischer, länglicher, pharmazeutisch wirksamer Arzneimitteltrockenpulverpartikel (42), von denen jedes eine Längsachse aufweist, bereitstellt und
die Partikel (42) auf einer Oberfläche des Substrats (32) abscheidet, indem man ein elektrisches Feld (36) bereitstellt, welches die Längsachse der Partikel (42) im wesentlichen lotrecht zu der Oberfläche des Substrats (32) ausrichtet, wobei eine Agglomeration der Partikel (42) und eine Haftung der Partikel (42) an dem Substrat (32) minimiert werden.

2. Verfahren nach Anspruch 1, welches Laden der Partikel (42) mit einer vorgegebenen Polarität und elektrostatisches Abscheiden der Partikel (42) auf dem Substrat (32) umfaßt.

3. Verfahren nach Anspruch 2, welches Bereitstellen der Partikel (42) mit einem solchen Aspektverhältnis, daß in jedem Partikel (42) entlang von dessen Längsachse ein elektrischer Dipol erzeugt wird, so daß ein elektrisches Feld (36) die Partikel (42) zu dem Substrat (32) anzieht und ausrichtet, umfaßt.

4. Verfahren nach Anspruch 1, wobei die Partikel (42) ein Aspektverhältnis von etwa 2:1 aufweisen.

5. Verfahren nach Anspruch 1, welches Bereitstellen eines Metallsubstrats (32) umfaßt.

6. Verfahren nach Anspruch 5, welches Bereitstellen eines Edelstahlsubstrats (32) umfaßt.

7. Verfahren nach Anspruch 1, welches Abscheiden einer kontrollierten Menge der Partikel (42) an jedem von mehreren vorherbestimmten Bereichen auf der Oberfläche des Substrats (32) unter Ausbildung einer Dosiseinheit an jedem vorherbestimmten Bereich umfaßt.

8. Verfahren nach Anspruch 1, welches die Stufe umfaßt, bei der man Partikelketten ausbildet, die im wesentlichen lotrecht zu dem Substrat (32) sind.

9. Verfahren nach Anspruch 1, welches Bereitstellen von Partikeln (42) mit einem Durchmessermaß im Bereich von etwa 0,5 bis 3 µm und einer Länge im Bereich von etwa 1 bis 10 µm umfaßt.

10. Verfahren nach Anspruch 1, welches die Stufe umfaßt, bei der man eine Vielzahl von Partikeln (42) an einer Vielzahl von getrennten, diskreten Stellen auf dem Substrat (32) bereitstellt, wobei jede Stelle eine Dosierung bildet.

11. Vorrichtung zum Abgeben eines pharmazeutisch aktiven Arzneimittels (18) mit einem Inhalator (2) mit einem Mundstück (8) und einem Arzneimittelraum (6) in Verbindung mit dem Mundstück (8) und
einem pharmazeutisch aktiven Trockenpulverarzneimittel (18), das auf einem Substrat (16) in dem Raum (6) für eine selektive Abgabe durch den Inhalator (2) abgeschieden ist, **dadurch gekennzeichnet, daß** das Arzneimittel (18) eine Vielzahl von dielektrischen, trockenen, länglichen Partikeln (42) umfaßt, die Partikel (42) solch ein Aspektverhältnis haben, daß in den Partikeln (42) ein elektrischer Dipol erzeugt wird, wenn sie einem elektrischen Feld (36) ausgesetzt werden, wobei die Partikel (42) mit ihren Längsachsen im wesentlichen lotrecht zu dem Substrat (16) auf dem Substrat abgeschieden werden und die Partikel von einem elektrischen Feld (36) zu dem Substrat (16) hin angezogen werden, um eine Agglomeration und eine Haftung der Partikel (42) an dem Substrat (16) zu minimieren.

12. Vorrichtung nach Anspruch 11, wobei das Aspektverhältnis etwa 2:1 beträgt.

13. Vorrichtung nach Anspruch 11, wobei die länglichen Partikel (42) jeweils gegenüberliegende Endspitzen aufweisen und eine Vielzahl der Partikel (42) das Substrat (16) mit einer der Partikelendspitzen berührt.

14. Vorrichtung nach Anspruch 11, welche eine Abgabeeinrichtung (12, 14) für das selektive Abgeben des Arzneimittels umfaßt.

15. Vorrichtung nach Anspruch 11, wobei eine erste Vielzahl der Partikel (42) mit einer zweiten Vielzahl der Partikel (42) in einem Spitze-zu-Spitze-Verhältnis in Eingriff ist.

16. Vorrichtung (30) zum Abscheiden von dielektrischen, länglichen Partikeln (42) eines pharmazeutisch wirksamen Arzneimitteltrockenpulvers auf einem Substrat (32) mit
einer Vielzahl länglicher, dielektrischer, trockener Partikel (42) eines pharmazeutisch wirksamen Arzneimittels, von denen jedes eine Längsachse aufweist, und
Mitteln zum Abscheiden der Partikel auf einer Oberfläche des Substrats (32), indem man ein elektrisches Feld (36) bereitstellt, welches die Längsachse der Partikel (42) im wesentlichen lotrecht zu der Substratoberfläche ausrichtet, wobei eine Agglomeration der Partikel (42) und eine Haftung der Partikel (42) an dem Substrat (32) minimiert werden.

17. Vorrichtung nach Anspruch 16, welche Mittel (34) zum Laden der Partikel (42) mit einer vorgegebenen Polarität und Mittel (36) für das elektrostatische Abscheiden der Partikel auf dem Substrat umfaßt.

18. Vorrichtung nach Anspruch 16, wobei die Partikel (42) ein solches Aspektverhältnis haben, daß in jedem Partikel (42) entlang von dessen Längsachse ein elektrischer Dipol erzeugt wird, und so, daß ein elektrisches Feld (36) die Partikel (42) elektrostatisch zu dem Substrat (32) hin anzieht.

19. Vorrichtung nach Anspruch 16, wobei die Partikel (42) ein Aspektverhältnis von etwa 2:1 aufweisen.

20. Vorrichtung nach Anspruch 16, wobei das Substrat (32) Metall ist.

21. Vorrichtung nach Anspruch 16, welche Partikelketten umfaßt, die im wesentlichen lotrecht zu dem Substrat (32) sind.

22. Vorrichtung nach Anspruch 16, wobei eine Vielzahl der Partikel (42) an jeder von einer Vielzahl getrennter, diskreter Stellen auf dem Substrat (32) unter Ausbildung einer Dosiseinheit an jeder Stelle abgeschieden ist.

## Revendications

1. Procédé pour déposer des particules (42) allongées diélectriques de poudre sèche de médicament actif pharmaceutique sur un substrat (32) comprenant les phases consistant à :
prévoir une pluralité de particules (42) allongées, diélectriques, sèches, de médicament actif pharmaceutique, chacune ayant un axe longitudinal ; et
déposer les particules (42) sur une surface du substrat (32) en créant un champ électrique (36) qui aligne l'axe longitudinal des particules (42) sensiblement perpendiculairement à la surface du substrat (32), de manière à rendre minimales l'agglomération des particules (42) et l'adhérence des particules (42) au substrat (32).

2. Procédé selon la revendication 1, comprenant le chargement des particules (42) avec une polarité donnée et le dépôt de manière électrostatique les particules (42) sur le substrat (32).

3. Procédé selon la revendication 2, comprenant la phase consistant à doter les particules (42) d'un rapport d'aspect de manière qu'un dipôle électrique soit créé dans chaque particule (42) le long de son axe longitudinal afin qu'un champ électrique (36) attire et aligne de manière électrostatique les particules (42) sur le substrat (32).

4. Procédé selon la revendication 1, dans lequel les particules (42) ont un rapport d'aspect d'environ 2:1.

5. Procédé selon la revendication 1, comprenant la phase consistant à prévoir un substrat (32) métallique.

6. Procédé selon la revendication 5, comprenant la phase consistant à prévoir un substrat (32) en acier inoxydable.

7. Procédé selon la revendication 1, comprenant le dépôt d'une quantité contrôlée des particules (42) à chacune d'une pluralité de régions prédéterminées sur la surface du substrat (32) pour former un dosage d'unité à chaque dite région prédéterminée.

8. Procédé selon la revendication 1, comprenant la phase de formation de chaînes de particules sensiblement perpendiculaires au substrat (32).

9. Procédé selon la revendication 1, comprenant la phase consistant à prévoir des particules (42) ayant une dimension diamétrale dans la plage d'environ 0,5 à 3 µm et une longueur dans la plage d'environ 1 à 10 µm.

10. Procédé selon la revendication 1, comprenant la phase consistant à prévoir une pluralité des particules (42) dans une pluralité d'emplacements discrets séparés sur le substrat (32), chaque emplacement constituant un dosage.

11. Dispositif pour distribuer un médicament actif pharmaceutique (18) comprenant :
un inhalateur (2) comportant un embout buccal (8) et une cavité de médicament (6) en communication avec l'embout buccal (8) ; et
un médicament actif pharmaceutique sous forme de poudre sèche (18) déposé sur un substrat (16) dans la cavité (6) pour une distribution sélective par l'inhalateur (2), **caractérisé en ce que** le médicament (18) comprend une pluralité de particules (42) allongées sèches diélectriques, les particules (42) ayant un rapport d'aspect de manière à créer un dipôle électrique dans les particules (42) lorsqu'elles sont exposées à un champ électrique (36) appliqué, les particules (42) étant déposées sur le substrat (16) avec leurs axes longitudinaux sensiblement perpendiculaires au substrat (16), les particules (42) étant attirées vers le substrat (16) par un champ électrique (36) pour rendre minimales l'agglomération et l'adhérence des particules (42) sur le substrat (16).

12. Dispositif selon la revendication 11, dans lequel le rapport d'aspect est d'environ 2:1.

13. Dispositif selon la revendication 11, dans lequel les particules (42) allongées comportent chacune des pointes d'extrémité opposées, une pluralité des particules (42) étant en contact avec le substrat (16) à l'une des pointes d'extrémité de particules.

14. Dispositif selon la revendication 11, comprenant des moyens de distribution (12, 14) pour une distribution sélective du médicament.

15. Dispositif selon la revendication 11, dans lequel une première pluralité des particules (42) engage une deuxième pluralité des particules (42) en une relation de pointe à pointe.

16. Dispositif (30) pour déposer des particules (42) allongées, diélectriques, de poudre sèche de médicament actif pharmaceutique sur un substrat (32) comprenant :
une pluralité de particules (42) allongées, diélectriques, sèches, de médicament actif pharmaceutique, chacune ayant un axe longitudinal ; et
un moyen pour déposer les particules sur une surface du substrat (32) en créant un champ électrique (36) qui aligne l'axe longitudinal des particules (42) sensiblement perpendiculairement à la surface du substrat, de manière à rendre minimales l'agglomération des particules (42) et l'adhérence des particules (42) au substrat (32).

17. Dispositif selon la revendication 16, comprenant un moyen (34) pour charger les particules (42) avec une polarité donnée et un moyen (36) pour déposer de manière électrostatique les particules sur le substrat.

18. Dispositif selon la revendication 16, dans lequel les particules (42) ont un rapport d'aspect de manière qu'un dipôle électrique soit créé dans chaque particule (42) le long de son axe longitudinal et de manière qu'un champ électrique (36) attire de manière électrostatique les particules (42) vers le substrat (32).

19. Dispositif selon la revendication 16, dans lequel les particules (42) ont un rapport d'aspect d'environ 2:1.

20. Dispositif selon la revendication 16, dans lequel le substrat (32) est en métal.

21. Dispositif selon la revendication 16, comprenant des chaînes de particules sensiblement perpendiculaires au substrat (32).

22. Dispositif selon la revendication 16, dans lequel une pluralité des particules (42) est déposée à chaque emplacement d'une pluralité d'emplacements discrets séparés sur le substrat (32), constituant un dosage d'unité à chaque emplacement.
